# EUROPEAN PATENT SPECIFICATION

(11) **EP 3 362 427 B1**
(45) Date of publication and mention of the grant of the patent: **04.08.2021**
(21) Application number: 16787662.2
(22) Date of filing: 11.10.2016
(51) Int. Cl.: C07C 41/03, C07C 43/13

(54) **PRODUCTION OF PROPYLENE GLYCOL MONOALKYL ETHER**
HERSTELLUNG VON PROPYLENGLYKOL-MONOALKYLETHER
PRODUCTION D'ÉTHER MONOALKYLIQUE DE PROPYLÈNE GLYCOL

(30) Priority: 12.10.2015 US 201514880653
(43) Date of publication of application: 22.08.2018
(73) Proprietor: Lyondell Chemical Technology, L.P., Houston, TX 77010 (US)
(72) Inventor: LI, Xiangmin, Houston, TX 77010 (US); CANDELA, Lawrence, M., Houston, TX 77010 (US); LEIPA, Mark, A., Houston, TX 77010 (US); LEYSHON, David, W., Houston, TX 77010 (US)
(74) Representative: LyondellBasell
(86) International application number: PCT/US2016/056448
(87) International publication number: WO 2017/066196

(56) References cited:
- WO-A1-2009/091379

## Description

### FIELD

This invention relates to a process for producing a propylene glycol monoalkyl ether.

### BACKGROUND

Propylene glycol monoalkyl ethers may be used as high-performance industrial solvents for paints and coatings, cleaners, inks, and a variety of other applications, including agricultural, cosmetic, electronic, textile, and adhesive products. They also may be used as chemical intermediates for end-products, such as propylene glycol ether acetates.

Typically, propylene glycol monoalkyl ethers are formed by the reaction of propylene oxide with an alcohol, such as methanol or 1-butanol. Although a catalyst is not required, the reaction is typically performed in the presence of a catalyst. A wide variety of catalysts and reaction conditions are taught in the prior art.

The catalysts used in this process include acidic, basic, and neutral species. Particularly useful catalysts include acids such as sulfuric acid, boric acid and some fluorine-containing acids; or bases such as alkali and alkaline earth metal hydroxides and alkoxides, tertiary amines, and certain metal oxides. G.B. Pat. No. 271,169, for instance, discloses the use of sulfuric acid, alkali metal alkoxides, and alkali metal salts of lower fatty acids. U.S. Pat. No. 2,327,053 teaches the use of metal halides such as stannic halides, antimony pentahalides, aluminum halides, zinc halides and ferric halides.

WO 2009/091379 discloses a process for producing a propylene glycol monoalkyl ether. The reaction and/or one or more of the distillation steps occur in the presence of an alkali metal borohydride. This document does not disclose a further distillation of the second bottoms stream and the introduction of an alkali metal borohydride into at least a portion of the resulting bottom stream.

A problem associated with these reactions, and in particular the use of alkali or alkaline earth metal alkoxide catalysts, is that the propylene glycol monoalkyl ether product may be contaminated with various carbonyl impurities (such as formaldehyde, acetaldehyde,
propionaldehyde, acetone, methoxy acetone, and methoxy butenone) that lead to high UV absorption. For particular applications, it may be necessary to limit the amount of carbonyl impurities and thus lower the UV absorbance of the propylene glycol monoalkyl ether product.

In sum, new processes to produce propylene glycol monoalkyl ethers are needed. Particularly useful processes will decrease the amount of carbonyl impurities and thus improve the UV absorbance and color of the propylene glycol monoalkyl ether product. We have discovered an effective, convenient process that produces propylene glycol monoalkyl ether having low UV absorbance.

### SUMMARY

The present disclosure generally includes processes for producing propylene glycol monoalkyl ether as described in claim 1..

One or more embodiments include the process of the preceding paragraph, wherein the alkali metal borohydride containing stream is introduced into the alkoxylation mixture.

One or more embodiments include the process of any preceding paragraph, wherein a concentration of caustic present in the at least a portion of the resulting bottoms stream is sufficient to substantially dissolve the alkali metal borohydride therein.

One or more embodiments include the process of any preceding paragraph, wherein the alcohol is selected from methanol, 1-propanol, 1-butanol, tert-butanol and combinations thereof.

One or more embodiments include the process of any preceding paragraph, wherein the alkali or alkaline earth metal alkoxide catalyst includes an alkali metal alkoxide.

One or more embodiments include the process of any preceding paragraph, wherein the alkali metal alkoxide catalyst includes a potassium alkoxide or a sodium alkoxide.

One or more embodiments include the process of any preceding paragraph, wherein the potassium alkoxide is selected from potassium methoxide, potassium n-propoxide, potassium n-butoxide, potassium t-butoxide and combinations thereof.

One or more embodiments include the process of any preceding paragraph, wherein the sodium alkoxide is selected from sodium methoxide, sodium n-propoxide, sodium n-butoxide, sodium t-butoxide and combinations thereof.

One or more embodiments include the process of any preceding paragraph, wherein the alkali metal borohydride includes sodium borohydride.

One or more embodiments include the process of any preceding paragraph, wherein the purified propylene glycol monoalkyl ether exhibits a UV absorbance, at 245 nm, of 1 or less.

One or more embodiments include the process of any preceding paragraph, wherein the second overhead stream includes at least 98 wt.% propylene glycol monoalkyl ether based on the total weight of the second overhead stream.

One or more embodiments include the process of any preceding paragraph, wherein the one or more distillations upstream of recovery of the second overhead stream occur in the presence of the alkali metal borohydride in a concentration in a range of 0.05 ppm to 1000 ppm.

One or more embodiments include the process of any preceding paragraph, wherein the one or more distillations upstream of recovery of the second overhead stream occur in the presence of the alkali metal borohydride in a concentration in a range of 0.1 ppm to 50 ppm.

One or more embodiments include the process of any preceding paragraph, wherein the alkoxylation mixture includes a component having a carbonyl functional group and the alkali metal borohydride reduces a concentration of the component having the carbonyl functional group.

### DETAILED DESCRIPTION

The process of the invention comprises first reacting a reaction mixture comprising propylene oxide and an alcohol in the presence of an alkali or alkaline earth metal alkoxide to produce an alkoxylation mixture comprising a propylene glycol monoalkyl ether (which may be referred to herein as "PGMA"). In this reaction, two propylene glycol monoalkyl ether isomers, 1-alkoxy-2-propanol and 2-alkoxy-1-propanol, may be produced. For instance the reaction of propylene oxide and methanol produces both 1-methoxy-2-propanol (known as "PM-1") and 2-methoxy-1-propanol ("PM-2"). PM-1, the major product from methanol propoxylation, is the isomer sold commercially.

The process to produce propylene glycol monoalkyl ethers is well known and propylene glycol monoalkyl ethers are commercially available products. Commercial products include Lyondell Chemical Company's ARCOSOLV® propylene glycol ethers, such as ARCOSOLV® PM (propylene glycol monomethyl ether), ARCOSOLV® PNB (propylene glycol normal butyl ether), ARCOSOLV® PTB (propylene glycol tertiary butyl ether), and ARCOSOLV® PNP (propylene glycol normal propyl ether).

The reaction mixture comprises propylene oxide and an alcohol. The alcohol used for the reaction is suitably an aliphatic, cycloaliphatic or an aromatic alcohol and may have one, two, or more hydroxyl groups. Preferably, the alcohol has only one hydroxyl group. The alcohol may be primary, secondary or tertiary in structure, and may be saturated or unsaturated as well as substituted with various substituents. Most preferably, the alcohol is a C₁-C₄ alcohol, particularly, methanol, ethanol, 1-propanol, 2-propanol, 1-butanol, and tert-butanol.

The relative amounts of propylene oxide and alcohol that make up the reaction mixture can vary over a fairly wide range. Usually, however, it is preferred to use at least one mole of alcohol for every propylene oxide equivalent. For example, when methanol reacts with propylene oxide to produce propylene glycol monomethyl ether, it is preferred to use 2 moles of methanol per mole of propylene oxide. Preferably, the molar ratio of alcohol to propylene oxide is at least 1.1:1 and is more preferably in the range from 1.5:1 to 5:1.

Although not necessary for the reaction, the reaction mixture may also include a solvent. Suitable solvents include C₅-C₂₀ aliphatic hydrocarbons such as hexane, C₆-C₂₀ aromatic hydrocarbons such as toluene, nitriles such as acetonitrile, and ethers such as methyl t-butyl ether.

The reaction of propylene oxide with an alcohol may be performed in the presence of an alkali or alkaline earth metal alkoxide catalyst. Although any alkali or alkaline earth metal alkoxide may be used (lithium, sodium, potassium, rubidium, cesium, magnesium, calcium, strontium, or barium alkoxides), alkali metal alkoxides are preferred. Potassium alkoxides and sodium alkoxides are particularly preferred. Preferred sodium alkoxides include sodium methoxide, ethoxide, n-propoxide, and n-butoxide, and t-butoxide. Preferred potassium alkoxides include potassium methoxide, ethoxide, n-propoxide, n-butoxide, and t-butoxide. Preferably, the alkoxide corresponds to the alcohol used in the reaction, that is, if the alcohol is methanol then the alkoxide is a methoxide such as sodium methoxide or potassium methoxide.

The reaction of propylene oxide and alcohol to form propylene glycol monoalkyl ether is preferably carried out at a temperature in the range of from 50°C to 250°C, more preferably from 100°C to 180°C. The reaction between propylene oxide and the alcohol is exothermic, so it may be desirable to apply cooling to the reaction mixture in order to control the reaction temperature. The reaction is preferably carried out at atmospheric pressure or at higher pressure up to (3000 psig) 20,786 MPa.

The reaction step of the invention includes batch, semi-batch, and continuous processes. In a typical batch reaction, the reactants (except for the catalyst) are charged to a reactor, catalyst is introduced, and the mixture is heated to the desired reaction temperature to form an alkoxylation mixture comprising propylene glycol monoalkyl ether. In a typical continuous reaction, streams of the propylene oxide, alcohol, any recycle streams, and alkali or alkaline earth metal alkoxide catalyst are fed continuously into a heated reaction zone to form an alkoxylation mixture comprising propylene glycol monoalkyl ether which is continuously withdrawn from the reactor.

Following reaction, the alkoxylation mixture is subjected to distillation in order to produce a propylene glycol monoalkyl ether product. The distillation steps include first distilling the alkoxylation mixture to produce a first overhead stream comprising propylene oxide and alcohol and a first bottoms stream comprising propylene glycol monoalkyl ether; and then distilling the first bottoms stream to produce purified propylene glycol monoalkyl ether as a second overhead stream.

The first distillation step, distilling the alkoxylation mixture, may be operated at any temperature and pressure which will afford a first distillation bottoms stream that contains a higher purity of propylene glycol monoalkyl ether than was contained in the alkoxylation mixture from the reaction. Prior to distillation, the alkoxylation mixture may be sent to a storage unit and/or a finishing drum in order to remove lights from the alkoxylation mixture, however such a storage unit or finishing drum is not necessary for the process of the invention. If the alkoxylation mixture is sent to a finishing drum, the system pressure in the finishing drum is reduced such that a significant amount of unreacted propylene oxide is flashed and vented from the alkoxylation mixture prior to distillation. This flashed propylene oxide may be recycled back to the reaction.

The second distillation step, distilling the first bottoms stream, may be operated at any temperature and pressure which will afford purified propylene glycol monoalkyl ether in the second overhead stream. The purified propylene glycol monoalkyl ether may be a mixture of 1-alkoxy-2-propanol (PM-1) and 2-alkoxy-1-propanol (PM-2). The purified propylene glycol monoalkyl ether may also be purified 1-alkoxy-2-propanol (PM-1), wherein any 2-alkoxy-1-propanol (PM-2) produced is separated from the second bottoms stream. As used herein, the term "purified propylene glycol monoalkyl ether" or "purified PGMA" refers to a process stream having at least 95 wt%, or at least 96 wt.%, or at least 97 wt.%, or at least 98 wt.%, or at least 99 wt.% propylene glycol monoalkyl ether based on the total weight of the process stream.

In general, the distillation towers (also referred to as columns) may be of conventional design. The towers are preferentially packed with conventional packing. The temperature and pressure in the towers may be adjusted depending on the type of propylene glycol monoalkyl ether produced.

The first distillation tower is used in order to remove alcohol and propylene oxide as an overhead stream and concentrate propylene glycol monoalkyl ether in the bottoms stream. Preferably, the first distillation tower is operated at a temperature of from 50°C to 200°C and a pressure of from (1 psig) 108 MPa to (150 psig) 1136 MPa.

The overhead from the first distillation tower (containing unreacted alcohol and propylene oxide) is preferentially recycled to the reaction step for further reaction. The first distillation bottoms, comprising propylene glycol monoalkyl ether, exits the first tower and is transferred to the second distillation tower for further purification of the propylene glycol monoalkyl ether.

In the second distillation tower, the first distillation bottoms stream is distilled. The overhead stream from the second distillation tower comprises high purity propylene glycol monoalkyl ether. In general, the second distillation tower is operated at a temperature of from 100° to 200°C and a pressure of from (1 mm Hg) 0.133 MPa to (760 mm Hg) 101.3 MPa.

In one or more embodiments, the second distillation bottoms stream, comprising heavier byproducts (such as dipropylene glycol ethers and tripropylene glycol ethers), and optionally 2-alkoxy-1-propanol (PM-2) where 1-alkoxy-2-propanol (PM-1) is purified in the overhead stream, is further distilled to form a resulting bottoms stream. As used herein, the term "heavier" is a relative term and relates to the boiling point of separated components. For example, in the second distillation, the heavier byproducts are those components that are less volatile, *i.e.,* have a higher boiling point, than those removed in the second overhead stream. Such further distillation may include introducing the second bottoms stream into a third distillation tower to form a third overhead stream including the PM-2 and a third bottoms stream including the heavier byproducts. The third distillation step, distilling the second bottoms stream, may be operated at any temperature and pressure which will afford PM-2 as the third overhead stream.

The third distillation bottoms stream may further be introduced into a fourth distillation tower to form a fourth overhead stream including dipropylene glycol ethers and a fourth bottoms stream (which may be the "resulting bottoms stream") including any remaining heavier byproducts. The fourth distillation step, distilling the third bottoms stream, may be operated at any temperature and pressure which will afford dipropylene glycol ethers as the fourth overhead stream.

Without the addition of an alkali metal borohydride to the reaction and/or distillation steps, the propylene glycol monoalkyl ether product typically contains by weight greater than 50 ppm of various carbonyl impurities and gives a UV absorbance (at 245 nm) of greater than 1. Typically, the propylene glycol monoalkyl ether produced with no alkali metal borohydride comprises by weight 50 to 2,000 ppm of various carbonyl impurities, usually 50 to 1,000 ppm.

Thus, the process of the invention requires that the reaction and/or one or more of the distillation steps occur in the presence of an alkali metal borohydride. The presence of alkali metal borohydride surprisingly reduces the carbonyl concentration and UV absorption in the propylene glycol monoalkyl ether product. Although any alkali metal borohydride may be used (lithium, sodium, potassium, rubidium, and cesium borohydride), sodium and lithium borohydride are preferred, and sodium borohydride is especially preferred.

If an alkali metal borohydride is added to the reaction mixture, the alkali metal borohydride is preferably added to the reaction mixture such that the reaction mixture comprises from 0.05 to 100 ppm alkali metal borohydride, more preferably from 0.1 to 50 ppm alkali metal borohydride.

If an alkali metal borohydride is added to the first distillation step, the alkali metal borohydride may be added into the distillation column as a separate stream from the alkoxylation mixture or may be added into the alkoxylation mixture prior to the first distillation step. Preferably, the alkali metal borohydride is added into the alkoxylation mixture prior to the first distillation step. The alkali metal borohydride is preferably added to the alkoxylation mixture such that the alkoxylation mixture comprises from 0.1 to 1,000 ppm alkali metal borohydride, more preferably from 1 to 100 ppm alkali metal borohydride.

If an alkali metal borohydride is added to the second distillation step, the alkali metal borohydride may be added into the distillation column as a separate stream from the first bottoms stream or may be added into the first bottoms stream prior to the second distillation step. Preferably, the alkali metal borohydride is added into the first bottoms stream prior to the second distillation step. The alkali metal borohydride is preferably added to the first bottoms stream such that the first bottoms stream comprises from 1 to 10,000 ppm alkali metal borohydride, more preferably from 1 to 100 ppm alkali metal borohydride.

Although an alkali metal borohydride may be added to the reaction step and both distillation steps, it is preferable to add alkali metal borohydride to the first and/or the second distillation step. It is especially preferred to add the alkali metal borohydride to the first distillation step.

In one or more embodiments, the alkali metal borohydride is introduced into at least a portion of the resulting bottoms stream, such as the fourth bottoms stream, for which the following non-limiting disclosure relates, to form an alkali metal borohydride containing stream. The fourth bottoms stream generally includes catalyst residual and thus caustic, which is capable of dissolving solid alkali metal borohydride therein. Furthermore, it has unexpectedly been found that the catalyst residual is capable of stabilizing the alkali metal borohydride. As a result, the alkali metal borohydride remains active for removal of carbonyl impurities rather than decomposing. The catalyst residual is capable of stabilizing the alkali metal borohydride, thus rendering it stable and active for a period of time sufficient to substantially remove carbonyl impurities to those levels designated herein.

In one or more embodiments, the alkali metal borohydride may be introduced into at least a portion of the fourth bottoms stream at a rate such that the alkali metal borohydride substantially dissolves (*i.e.,* to within 1 wt.%) in the at least a portion of the fourth bottoms stream and is easily introduced back into the distillation process, preferably upstream of the product recovery (*i.e.,* the purified PGMA recovered in the second overhead stream). In one or more embodiments, the alkali metal borohydride is introduced into a portion of the fourth bottoms stream. For example, the portion of the fourth bottoms stream used for the borohydride solution may include at least 2% or from 2% to 80%, or from 10% to 40% , or from 5% to 20% of the fourth bottoms stream.

In an alternative embodiment, the alkali metal borohydride is introduced into the entire portion of the fourth bottoms stream. It is contemplated that the alkali metal borohydride may be introduced into at least a portion of the third bottoms stream. However, such introduction is at a rate sufficient to substantially dissolve the alkali metal borohydride in the stream. In order for the alkali metal borohydride to dissolve therein, it is contemplated that the stream in which it is introduced (*e.g*., the third bottoms stream and/or the fourth bottoms stream) has a concentration of caustic sufficient to achieve such dissolution. Furthermore, the stream in which the alkali metal borohydride is introduced, as well as the portion of that stream which is utilized, should be such that when re-introduced into the process, the salt content of the fourth bottoms stream is not greatly increased (*e.g*., not by more than 0.5 wt.% absolute, or 20 wt.% relative to the initial salt content of the stream).

Thus, one or more embodiments include recycling at least a portion of the fourth bottoms stream including the alkali metal borohydride to the first distillation step, the second distillation step or a combination thereof. The at least a portion of the fourth bottoms stream including the alkali metal borohydride introduced into the upstream distillation step is such that alkali metal borohydride concentration introduced into the distillation is equivalent to that discussed previously herein. In one or more embodiments, a concentration of alkali metal borohydride present of the at least a portion of the fourth bottoms stream may include less than 1 wt.% alkali metal borohydride or from 0.01 wt.% to 1 wt.%, or from 0.05 wt.% to 0.95 wt.%, or from 0.1 wt.% to 0.8 wt.%, or from 0.5 wt.% to 0.75 wt.% based on the total weight of the at least a portion of the fourth bottoms stream, for example.

The choice of introduction location is such that sufficient reaction time is provided to reduce the carbonyl impurities such that the UV absorbence of the purified PGMA is at levels recited herein.

Following the process of the invention, a purified propylene glycol monoalkyl ether product having a decreased carbonyl impurities content is produced. Preferably, the purified propylene glycol monoalkyl ether product has a UV absorbance, at 245 nm, of 1 or less.

### EXAMPLES

The following examples merely illustrate the invention. Those skilled in the art will recognize many variations that are within the spirit of the invention and scope of the claims.

### EXAMPLE 1: REACTION RUNS

Comparison Run 1A: Methanol (132 g), propylene oxide (110 g), and a 2-methoxy-1-propanol recycle mixture (60 g) are added to a stainless steel reactor and the temperature of the reaction mixture is raised to 130°C. A potassium methoxide catalyst mixture (1.27 g, of 25 wt.% KOCH₃ in methanol) is added to the reaction mixture and stirred. Samples are taken from the reaction mixture periodically (at 1 h and 10 min and at 2 hr and 10 min after catalyst addition) and analyzed for carbonyl content and UV absorbance. Results are shown in Table 1.

Run 1B: Run 1B is run according to the procedure of Comparison Run 1A with the exception that NaBH₄ is added to the reaction mixture with the methanol, resulting in a 0.3 ppm NaBH₄ concentration in the reaction mixture. Results are shown in Table 1.

The results show that a small amount of sodium borohydride in the reaction mixture leads to a reduced amount of carbonyl impurities and reduced UV absorbance.

### EXAMPLE 2: BATCH DISTILLATION RUNS

Comparison Run 2A: A 1-methoxy-2-propanol reaction solution (1198.3 g, containing 778.19 g PM-1) is distilled under vacuum using an 8 mm I.D. spinning band batch distillation column (B/R Instrument Corporation, Easton MD). The condenser pressure is controlled at 368 mm Hg (49 MPa) and the reflux ratio is set at 50 to 1. This results in a boiling temperature of 105°C in the distillation reboiler pot. Samples of the overhead, purified PM-1 are taken in 20 mL increments and analyzed for carbonyl content and UV absorbance (at 245 nm). Results are shown in Table 2.

Run 2B: Run 2B is run according to the procedure of Comparison Run 2A with the exception that 200 ppm NaBH₄ (0.24 g) is added to the 1-methoxy-2-propanol solution (1200 g, containing 779.29 g PM-1). Results are shown in Table 2.

The results show that the addition of sodium borohydride into the batch distillation pot leads to a reduced amount of carbonyl impurities and reduced UV absorbance for the first 40 mL of PM-1 collected overhead, until the sodium borohydride in the pot was depleted after 7 hours at 105°C.

### EXAMPLE 3: CONTINUOUS DISTILLATION RUNS

Comparison Run 3A: A 1-methoxy-2-propanol reaction solution, containing 65 wt.% PM-1, is purified by continuous distillation under vacuum using a distillation column (1 in. I.D. x 4 ft) containing PRO-PAK® packing. The 1-methoxy-2-propanol reaction solution is fed into the distillation column at (18 inches) 42.72 cm from the bottom of the column and the temperature at the feeding tray is maintained at 118-119°C during the distillation procedure. Feed rate is adjusted to reach a desired liquid residence time. The reboiler liquid volume is controlled at 220 mL and pot temperature is maintained at 121-122°C. The reflux ratio is controlled at 5 to 1. Purified PM-1 product is withdrawn continuously overhead. PM-1 product samples are analyzed for carbonyl content and UV absorbance (at 245 nm). Results are shown in Table 3.

Run 3B: Run 3B is run according to the procedure of Comparison Run 3A with the exception that 42 ppm NaBH₄ is added to the 1-methoxy-2-propanol reaction solution. Results are shown in Table 3.

The results show that the addition of sodium borohydride into the continuous distillation reduces both carbonyl impurities and UV absorbance for the PM-1 product collected overhead.

**TABLE 1: Reaction Run Data**

| **Run #** | **Sample Time** | **Carbonyl Amount (ppm)** | **UV, abs.** |
|---|---|---|---|
| 1A* | 1:10 | 28 | 1.05 |
| 1B | 1:10 | 26 | 0.87 |
| | | | |
| 1A* | 2:10 | 36 | 1.64 |
| 1B | 2:10 | 30 | 1.3 |

| | | | |
|---|---|---|---|
| * Comparative Example | | | |

**TABLE 2: Distillation Run Data**

| **Run #** | **PM-1 Collected Overhead (mL)** | **Carbonyl Amount (ppm)** | **UV, abs.** |
|---|---|---|---|
| 2A* | 20 | 1209 | 3.87 |
| 2B | 20 | 10 | 0.16 |
| | | | |
| 2A* | 40 | 609 | 3.21 |
| 2B | 40 | 8 | 0.15 |
| | | | |
| 2A* | 60 | 351 | 2.66 |
| 2B | 60 | 388 | 0.86 |

| | | | |
|---|---|---|---|
| * Comparative Example | | | |

**TABLE 3: Distillation Run Data**

| **Run #** | **Residence Time (minute)** | **Carbonyl Amount (ppm)** | **UV, abs.** |
|---|---|---|---|
| 3A* | 115 | 58 | 0.96 |
| 3B | 119 | 6 | 0.16 |
| | | | |
| 3A* | 59 | 54 | 0.96 |
| 3B | 68 | 9 | 0.16 |
| | | | |
| 3A* | 42 | 76 | 1.01 |
| 3B | 46 | 21 | 0.21 |

| | | | |
|---|---|---|---|
| * Comparative Example | | | |

## Claims

1. A process for producing propylene glycol monoalkyl ether comprising:
contacting propylene oxide and an alcohol in the presence of an alkali or alkaline earth metal alkoxide catalyst to produce an alkoxylation mixture comprising propylene glycol monoalkyl ether;
distilling the alkoxylation mixture at a temperature of from 50 °C to 200 °C and a pressure of from 6.9 KPa to 1034.2 KPa to produce a first overhead stream comprising propylene oxide and the alcohol and a first bottoms stream comprising propylene glycol monoalkyl ether;
distilling the first bottoms stream to produce a second overhead stream comprising purified propylene glycol monoalkyl ether and a second bottoms stream comprising heavier byproducts;
further distilling the second bottoms stream produce a third overhead stream comprising 2-methoxy-1-propanol and a third bottoms stream comprising dipropylene glycol ether;
distilling the third bottoms stream to produce a fourth overhead stream comprising dipropylene glycol ether and the resulting bottoms stream comprising caustic and heavier byproducts;
introducing a solid alkali metal borohydride into at least a portion of the resulting bottoms stream to form an alkali metal borohydride containing stream; and
introducing the alkali metal borohydride containing stream into one or more distillations upstream of recovery of the second overhead stream.

2. The process of claim 1, wherein the alkali metal borohydride containing stream is introduced into the alkoxylation mixture.

3. The process of claim 1, wherein a concentration of caustic present in the at least a portion of the resulting bottoms stream is sufficient to dissolve the solid alkali metal borohydride therein.

4. The process of claim 1, wherein the alcohol is selected from methanol, 1-propanol, 1-butanol, tert-butanol and combinations thereof.

5. The process of claim 1, wherein the alkali or alkaline earth metal alkoxide catalyst comprises an alkali metal alkoxide.

6. The process of claim 6, wherein the alkali metal alkoxide catalyst comprises a potassium alkoxide or a sodium alkoxide.

7. The process of claim 7, wherein the potassium alkoxide is selected from potassium methoxide, potassium n-propoxide, potassium n-butoxide, potassium t-butoxide and combinations thereof.

8. The process of claim 7, wherein the sodium alkoxide is selected from sodium methoxide, sodium n-propoxide, sodium n-butoxide, sodium t-butoxide and combinations thereof.

9. The process of claim 1, wherein the alkali metal borohydride comprises sodium borohydride.

10. The process of claim 1, wherein the purified propylene glycol monoalkyl ether exhibits a UV absorbance, at 245 nm, of 1 or less.

11. The process of claim 1, wherein the second overhead stream comprises at least 98 wt.% propylene glycol monoalkyl ether based on the total weight of the second overhead stream.

12. The process of claim 1, wherein the one or more distillations upstream of recovery of the second overhead stream occur in the presence of the alkali metal borohydride in a concentration in a range of 0.05 ppm to 1000 ppm.

13. The process of claim 1, wherein the one or more distillations upstream of recovery of the second overhead stream occur in the presence of the alkali metal borohydride in a concentration in a range of 0.1 ppm to 50 ppm.

14. The process of claim 1, wherein the alkoxylation mixture comprises a component having a carbonyl functional group and the alkali metal borohydride reduces a concentration of the component having the carbonyl functional group.

## Patentansprüche

1. Verfahren zur Herstellung von Propylenglykol-Monoalkylether, umfassend:
Kontaktieren von Propylenoxid und einem Alkohol in Gegenwart von Alkali- oder Erdalkalimetallalkoxidkatalysator, um eine Alkoxylierungsmischung zu produzieren, die Propylenglykol-Monoalkylether umfasst;
Destillieren der Alkoxylierungsmischung bei einer Temperatur von 50 °C bis 200 °C und einem Druck von 6,9 KPa bis 1034,2 KPa, um einen ersten Kopfproduktstrom, der Propylenoxid und den Alkohol umfasst, und einen ersten Sumpfproduktstrom zu produzieren, der Propylenglykol-Monoalkylether umfasst;
Destillieren des ersten Sumpfproduktstroms, um einen zweiten Kopfproduktstrom, der gereinigten Propylenglykol-Monoalkylether umfasst, und einen zweiten Sumpfproduktstrom zu produzieren, der schwerere Nebenprodukte umfasst;
weiteres Destillieren des zweiten Sumpfproduktstroms, um einen dritten Kopfproduktstrom, der 2-Methoxy-1-propanol umfasst, und einen dritten Sumpfproduktstrom zu produzieren, der Dipropylenglykolether umfasst;
Destillieren des dritten Sumpfproduktstroms, um einen vierten Kopfproduktstrom zu produzieren, der Dipropylenglykolether umfasst, und wobei der resultierende Sumpfproduktstrom Ätzmittel und schwerere Nebenprodukte umfasst;
Einbringen von festem Alkalimetallborhydrid in mindestens einen Anteil des resultierenden Sumpfproduktstroms, um einen Alkalimetallborhydrid enthaltenden Strom zu bilden; und
Einbringen des Alkalimetallborhydrid enthaltenden Stroms in eine oder mehrere Destillationen vorgeordnet zu der Gewinnung des zweiten Kopfproduktstroms.

2. Verfahren nach Anspruch 1, wobei der Alkalimetallborhydrid enthaltende Strom in die Alkoxylierungsmischung eingebracht wird.

3. Verfahren nach Anspruch 1, wobei eine Konzentration an Ätzmittel, die in dem mindestens einen Anteil des resultierenden Sumpfproduktstroms vorhanden ist, ausreicht, um das feste Alkalimetallborhydrid darin zu lösen.

4. Verfahren nach Anspruch 1, wobei der Alkohol ausgewählt ist aus Methanol, 1-Propanol, 1-Butanol, tert.-Butanol und Kombinationen davon.

5. Verfahren nach Anspruch 1, wobei der Alkali- oder Erdalkalimetallalkoxidkatalysator ein Alkalimetallalkoxid umfasst.

6. Verfahren nach Anspruch 6, wobei der Alkalimetallalkoxidkatalysator ein Kaliumalkoxid oder ein Natriumalkoxid umfasst.

7. Verfahren nach Anspruch 7, wobei das Kaliumalkoxid ausgewählt ist aus Kaliummethoxid, Kalium-n-propoxid, Kalium-n-butoxid, Kalium-t-butoxid und Kombinationen davon.

8. Verfahren nach Anspruch 7, wobei das Natriumalkoxid ausgewählt ist aus Natriummethoxid, Natrium-n-propoxid, Natrium-n-butoxid, Natrium-t-butoxid und Kombinationen davon.

9. Verfahren nach Anspruch 1, wobei das Alkalimetallborhydrid Natriumborhydrid umfasst.

10. Verfahren nach Anspruch 1, wobei der gereinigte Propylenglykol-Monoalkylether eine UV-Extinktion bei 245 nm von 1 oder weniger zeigt.

11. Verfahren nach Anspruch 1, wobei der zweite Kopfproduktstrom mindestens 98 Gew.% Propylenglykol-Monoalkylether umfasst, bezogen auf das Gesamtgewicht des zweiten Kopfproduktstroms.

12. Verfahren nach Anspruch 1, wobei die eine oder mehreren Destillationen vorgeordnet zu der Gewinnung des zweiten Kopfproduktstroms in Gegenwart des Alkalimetallborhydrids in einer Konzentration in einem Bereich von 0,05 ppm bis 1000 ppm stattfinden.

13. Verfahren nach Anspruch 1, wobei die eine oder mehreren Destillationen vorgeordnet zu der Gewinnung des zweiten Kopfproduktstroms in Gegenwart des Alkalimetallborhydrids in einer Konzentration in einem Bereich von 0,1 ppm bis 50 ppm stattfinden.

14. Verfahren nach Anspruch 1, wobei die Alkoxylierungsmischung eine Komponente mit einer funktionalen Carbonylgruppe umfasst und das Alkalimetallborhydrid eine Konzentration der Komponente mit der funktionalen Carbonylgruppe reduziert.

## Revendications

1. Procédé de production de propylèneglycol monoalkyl éther comprenant :
la mise en contact d'oxyde de propylène et d'un alcool en présence d'un catalyseur de type alcoxyde de métal alcalin ou alcalino-terreux pour produire un mélange d'alcoxylation comprenant du propylèneglycol monoalkyl éther ;
la distillation du mélange d'alcoxylation à une température de 50°C à 200°C et à une pression de 6,9 KPa à 1034,2 KPa pour produire un premier flux de tête comprenant de l'oxyde de propylène et l'alcool et un premier flux de fond comprenant du propylèneglycol monoalkyl éther ;
la distillation du premier flux de fond pour produire un deuxième flux de tête comprenant du propylèneglycol monoalkyl éther purifié et un deuxième flux de fond comprenant des sous-produits plus lourds ;
la distillation supplémentaire du deuxième flux de fond pour produire un troisième flux de tête comprenant du 2-méthoxy-1-propanol et un troisième flux de fond comprenant du dipropylèneglycol éther ;
la distillation du troisième flux de fond pour produire un quatrième flux de tête comprenant du dipropylèneglycol éther et le flux de fond résultant comprenant du caustique et des sous-produits plus lourds ;
l'introduction d'un borohydrure de métal alcalin solide dans au moins une partie du flux de fond résultant pour former un flux contenant du borohydrure de métal alcalin ; et
l'introduction du flux contenant du borohydrure de métal alcalin dans une ou plusieurs distillations en amont de la récupération du deuxième flux de tête.

2. Procédé selon la revendication 1, le flux contenant du borohydrure de métal alcalin étant introduit dans le mélange d'alcoxylation.

3. Procédé selon la revendication 1, une concentration en caustique présente dans ladite au moins une partie du flux de fond résultant étant suffisante pour dissoudre le borohydrure de métal alcalin solide en son sein.

4. Procédé selon la revendication 1, l'alcool étant choisi parmi le méthanol, le 1-propanol, le 1-butanol, le tert-butanol et des combinaisons de ceux-ci.

5. Procédé selon la revendication 1, le catalyseur de type alcoxyde de métal alcalin ou alcalino-terreux comprenant un alcoxyde de métal alcalin.

6. Procédé selon la revendication 6, le catalyseur d'alcoxyde de métal alcalin comprenant un alcoxyde de potassium ou un alcoxyde de sodium.

7. Procédé selon la revendication 7, l'alcoxyde de potassium étant choisi parmi le méthoxyde de potassium, le n-propoxyde de potassium, le n-butoxyde de potassium, le t-butoxyde de potassium et des combinaisons de ceux-ci.

8. Procédé selon la revendication 7, l'alcoxyde de sodium étant choisi parmi le méthoxyde de sodium, le n-propoxyde de sodium, le n-butoxyde de sodium, le t-butoxyde de sodium et des combinaisons de ceux-ci.

9. Procédé selon la revendication 1, le borohydrure de métal alcalin comprenant du borohydrure de sodium.

10. Procédé selon la revendication 1, le propylèneglycol monoalkyl éther purifié présentant une absorbance UV, à 245 nm, de 1 ou moins.

11. Procédé selon la revendication 1, le deuxième flux de tête comprenant au moins 98 % en poids de propylèneglycol monoalkyl éther sur la base du poids total du deuxième flux de tête.

12. Procédé selon la revendication 1, ladite une ou lesdites plusieurs distillations en amont de la récupération du deuxième flux de tête se produisant en présence du borohydrure de métal alcalin en une concentration dans une plage de 0,05 ppm à 1000 ppm.

13. Procédé selon la revendication 1, ladite une ou lesdites plusieurs distillations en amont de la récupération du deuxième flux de tête se produisant en présence du borohydrure de métal alcalin en une concentration dans une plage de 0,1 ppm à 50 ppm.

14. Procédé selon la revendication 1, le mélange d'alcoxylation comprenant un constituant présentant un groupe fonctionnel carbonyle et le borohydrure de métal alcalin réduisant une concentration en constituant présentant le groupe fonctionnel carbonyle.
